# DEMANDE DE BREVET EUROPEEN

(11) **EP 1 523 937 A1**
(43) Date de publication de la demande: **20.04.2005**
(21) Numéro de dépôt: 04292459.7
(22) Date de dépôt: 15.10.2004
(51) Int. Cl.: A61B 5/103

(54) **Ensemble comportant un système d'analyse du niveau de clarté de la peau et des gammes de produits cosmétiques**

(30) Priorité: 16.10.2003 FR 0312104
(71) Demandeur: L'OREAL, 75008 Paris (FR)
(72) Inventeur: Caisey, Laurence, 94320 Thiais (FR)
(74) Mandataire: Tanty, François

(57) **Abrégé**

La présente invention concerne un ensemble comportant :
- une pluralité de gammes de produits cosmétiques, chaque gamme regroupant un ensemble de produits associés à un même niveau de clarté de la peau, ce niveau de clarté étant repéré par un identifiant (5),
- un système d'analyse (1) d'au moins une image de la peau, agencé pour délivrer un identifiant (5) représentatif du niveau de clarté de la peau analysée, cet identifiant étant l'un de ceux desdites gammes de produits.

## Description

La présente invention concerne le domaine du maquillage et du soin.

Il peut s'avérer difficile aujourd'hui pour un consommateur de choisir un produit cosmétique, notamment de maquillage, adapté au teint de sa peau, en raison de la grande diversité de l'offre.

L'invention vise notamment à faciliter le choix d'un produit cosmétique adapté au teint du consommateur.

L'invention répond à ce besoin grâce à un ensemble comportant :
- une pluralité de gammes de produits cosmétiques, chaque gamme regroupant un ensemble de produits associés à un même niveau de clarté de la peau, ce niveau de clarté étant repéré par un identifiant,
- un système d'analyse d'au moins une image de la peau, agencé pour délivrer un identifiant représentatif du niveau de clarté de la peau analysée, cet identifiant étant l'un de ceux desdites gammes de produits.

Par « produit cosmétique », on désigne notamment les produits tels que définis dans la Directive 93/35 CEE du Conseil du 14 juin 1993, modifiant, pour la sixième fois, la Directive 76/768/CEE concernant le rapprochement des législations des états membres relatives aux produits cosmétiques.

Les produits cosmétiques comprennent ainsi les produits destinés au maquillage de la peau, des phanères et des muqueuses et les produits de soin destinés par exemple à diminuer les signes du vieillissement et protéger ou hydrater la peau.

Par « délivrer », il faut comprendre l'émission d'un message visuel et/ou sonore, par exemple l'affichage sur un afficheur à LEDS ou écran de type tube cathodique, à cristaux liquides ou autre, d'une information, l'impression d'une information, la diffusion au moyen d'un haut-parleur sur le lieu de l'analyse, ou l'envoi d'une information à distance, notamment par messagerie téléphonique ou informatique ou courrier physique.

Par « identifiant », on désigne tout signe pouvant servir à repérer le niveau de clarté, par exemple un ou plusieurs caractères d'écriture, notamment alphanumériques, un ou plusieurs pictogrammes, une ou plusieurs couleurs, un ou plusieurs reliefs ou encore une forme de conditionnement particulière. L'identifiant n'a pas nécessairement à figurer un emballage du produit. Il peut figurer par exemple sur un support de type papier ou logiciel, notamment sur une page écran, en association avec les références commerciales du produit associé au niveau de clarté correspondant.

Grâce à l'invention, le choix d'un produit de maquillage adapté au teint est facilité puisque le consommateur connaissant son niveau de clarté ou une personne conseillant le consommateur peut sélectionner ce produit parmi le nombre restreint de produits composant la gamme correspondante, ce qui procure un gain de temps appréciable et une fiabilité complémentaire, le risque de choisir un produit de maquillage ou de soin ne convenant pas étant réduit, voire supprimé.

Le niveau de clarté n'est pas nécessairement exprimé en relation avec un espace colorimétrique de référence, par exemple l'espace colorimétrique CIE 1976 (L*a*b*). Le niveau de clarté peut, le cas échéant, être associé à une plage de valeurs de luminosité psychométrique dans l'espace colorimétrique CIE 1976 (L*a*b*).

Le système d'analyse peut être réalisé sous diverses formes, en fonction notamment de l'emplacement où a lieu l'analyse, par exemple un point de vente, un salon de beauté ou chez le consommateur.

Le système d'analyse comporte avantageusement une caméra, de préférence une caméra couleur, permettant d'acquérir au moins une image de la peau avec un grossissement optique.

Le facteur de grossissement peut être compris entre 3 et 15 par exemple.

La caméra couleur peut être agencée pour délivrer des signaux rouge, vert et bleu.

La caméra comporte avantageusement une source d'éclairage intégrée, notamment une source de lumière blanche, et elle peut être agencée par exemple de manière à pouvoir être appliquée contre la peau lors de l'acquisition d'une image, ce qui facilite la mise au point.

La caméra peut être une caméra à faible coût.

La caméra peut être reliée au reste du système d'analyse par une liaison filaire ou non. L'image peut, le cas échéant, être transmise au reste du système d'analyse par réseau téléphonique ou informatique.

De préférence, la caméra est sans balance du blanc automatique, ou lorsqu'une telle balance est présente, elle est désactivée.

Dans un exemple de mise en oeuvre de l'invention, le système d'analyse comporte des moyens agencés pour convertir, le cas échéant, l'image délivrée par la caméra en une image monochrome, notamment une image vert et noir, par exemple grâce au signal de vert et déterminer la luminosité moyenne (c'est-à-dire la quantité relative de lumière) d'une partie au moins de l'image monochrome. Une caméra monochrome pourrait encore être utilisée.

Le système d'analyse peut notamment comporter un micro-ordinateur équipé d'une carte d'acquisition graphique et être programmé de manière à calculer la luminosité moyenne à partir d'une image au moins transmise par la caméra. Ce calcul peut se faire en temps réel.

Le système d'analyse peut être agencé pour affecter à la valeur de luminosité moyenne ainsi calculée un niveau de clarté, en utilisant par exemple une table de correspondance, dans laquelle à chaque niveau de clarté est associée une plage de valeur de luminosité moyenne.

La luminosité moyenne peut être calculée sur tout ou partie d'au moins une image, et notamment peut être calculée après acquisition d'une pluralité d'images, en calculant pour chacune d'elles la luminosité moyenne et en faisant la moyenne des valeurs moyennes ainsi obtenues.

Le nombre de niveaux de clarté est supérieur à 2, et par exemple est compris entre 3 et 25, avantageusement compris entre 6 et 12, par exemple égal à 9.

Le système d'analyse peut le cas échéant être agencé en outre pour délivrer une information relative à la tonalité de couleur de la peau, par exemple une indication matérialisant la tonalité plutôt jaune ou plutôt rouge de la peau.

Cette indication peut être présentée par exemple à côté de l'identifiant de niveau de clarté, sur chaque produit desdites gammes ou sur un support contenant leurs références, notamment commerciales.

Selon un autre aspect de l'invention, l'ensemble comporte au moins un support destiné à faciliter le choix d'un produit, connaissant le niveau de clarté de la peau.

Ce support peut par exemple se présenter sous forme « papier » ou logiciel.

Le support peut être agencé de manière à permettre au consommateur de visualiser une pluralité de pages contenant des informations associées aux produits desdites gammes.

Dans le cas d'un support papier, celui-ci peut se présenter par exemple sous la forme d'une brochure comportant des feuilles reliées entre elles et des onglets sur lesquels sont imprimés les différents identifiants, ces onglets permettant d'accéder rapidement aux produits de la gamme correspondant à l'identifiant concerné.

Le support peut encore être agencé pour permettre de visualiser les effets d'un maquillage obtenu avec des produits donnés d'une gamme.

Le support peut notamment comporter des volets qui correspondent à des parties respectives du visage et qui permettent de modifier l'aspect du maquillage d'une partie du visage tout en laissant le maquillage inchangé pour la ou les autres parties du visage correspondant aux autres volets.

Le support peut par exemple comporter des volets correspondant à des demi-visages, notamment des volets supérieurs correspondant au maquillage des yeux et des volets inférieurs correspondant au maquillage des lèvres et des joues.

Pour un niveau de clarté au moins, le support peut comporter au moins deux volets successifs correspondant à la même partie du visage, chacun de ces volets comportant au recto une image du visage et au verso des informations concernant des produits de maquillage. Les volets peuvent être agencés de telle sorte que les produits référencés sur le verso d'un volet correspondent au résultat de maquillage présenté sur le recto du volet immédiatement suivant.

Un tel support permet au consommateur de voir le résultat de différentes combinaisons de maquillage du haut du visage et du bas du visage, tout en pouvant identifier rapidement les produits permettant d'obtenir un tel maquillage.

Un résultat similaire peut être obtenu lorsque le support est un logiciel programmé pour permettre l'affichage simultané sur une même page écran d'une simulation de maquillage et du ou des produits utilisés pour cette simulation.

Dans le cas d'un logiciel, celui-ci peut en outre être adapté de manière à ce que la simulation du maquillage ait lieu sur la propre image du consommateur, après acquisition de cette dernière au moyen d'une caméra, d'un scanner ou de la réception d'un fichier informatique.

Les informations concernant les produits, qui figurent sur le support, peuvent notamment comporter un échantillon de la couleur du produit, un numéro de référence et/ou la marque du produit, la nature de la surface destinée à recevoir ce produit, notamment la nature de la peau lorsque le produit est destiné à être appliqué sur la peau. Le support peut notamment indiquer pour un produit au moins le type de peau auquel ce produit est destiné, à savoir peau sèche, peau normale, peau normale à mixte, tous types de peau, peau mixte et/ou le cas échéant, indiquer si le produit procure un effet anti-âge.

Le support peut également comporter des informations liées à la forme galénique du produit, et indiquer notamment si celui-ci se présente sous une forme liquide ou solide, notamment pulvérulente ou compacte.

Le support peut encore comporter des informations liées à la tonalité de couleur que permet d'apporter le produit, par exemple un maquillage de type bronzé, ton sur ton ou éclairci.

Chaque gamme de produits peut comporter des produits destinés à être appliqués sur la peau, comme des fonds de teint, ombres à paupières, eye-liner, blush, des produits destinés à être appliqués sur les muqueuses tels qu'un rouge à lèvres, crayon à lèvres, des produits destinés à être appliqués sur les cils ou les sourcils tels qu'un mascara, un crayon à sourcils, un crayon khôl, et des produits destinés à être appliqués sur les ongles tels qu'un vernis à ongles.

L'invention a encore pour objet, selon un autre de ses aspects, un système d'analyse de la clarté de la peau, comportant :
- une caméra permettant d'acquérir au moins une image de la peau,
- des moyens de traitement d'au moins une image acquise par la caméra, agencés pour délivrer un identifiant associé au niveau de clarté mesuré, cet identifiant figurant par ailleurs
   - sur des produits d'une pluralité de gammes de produits associées respectivement aux différents niveaux de clarté susceptibles d'être délivrés par les moyens de traitement, ou
   - sur un support contenant des références de produits d'une pluralité de gammes de produits associées respectivement aux différents niveaux de clarté susceptibles d'être délivrés par les moyens de traitement.

L'invention a encore pour objet, selon un autre de ses aspects, un procédé de traitement cosmétique, notamment de maquillage de la peau, des phanères ou des muqueuses, comportant les étapes suivantes :
- associer à un consommateur un identifiant permettant de repérer le niveau de clarté de sa peau,
- en fonction de cet identifiant, sélectionner au moins un produit cosmétique parmi un ensemble de gammes de produits repérées par des identifiants de niveau de clarté,
- traiter, notamment maquiller, le consommateur avec le ou les produits sélectionnés.

L'invention a encore pour objet un procédé pour fournir un conseil en matière de beauté, comportant les étapes suivantes :
- associer à un consommateur un identifiant permettant de repérer le niveau de clarté de sa peau,
- en fonction de cet identifiant, conseiller au moins un produit cosmétique parmi un ensemble de gammes de produits repérées par des identifiants de niveau de clarté.

L'invention a encore pour objet un procédé de fabrication d'un ensemble de gammes de produits cosmétiques, notamment des produits destinés au maquillage de la peau, des muqueuses et des phanères, comportant l'apposition sur chaque produit de la gamme d'un identifiant correspondant à un niveau de clarté de la peau pour lequel ce produit est adapté.

Cet identifiant peut par exemple être un signe figurant sur une étiquette collée sur un récipient contenant le produit ou imprimé sur celui-ci.

L'invention a encore pour objet un procédé de fabrication d'un ensemble de gammes de produits cosmétiques, notamment des produits destinés au maquillage de la peau, des muqueuses et des phanères, comportant l'attribution à chaque produit de la gamme d'un identifiant correspondant à un niveau de clarté de la peau pour lequel ce produit est adapté. L'attribution de l'identifiant peut se faire par exemple en portant au moins une référence, par exemple commerciale, du produit dans une table en correspondance avec un identifiant de niveau de clarté. La table peut être un document de type papier ou un ficher informatique.

L'invention pourra être mieux comprise à la lecture de la description détaillée qui va suivre, d'exemples de mise en oeuvre non limitatifs de celle-ci, et à l'examen du dessin annexé, qui fait partie intégrante de la description, sur lequel :
- la figure 1 représente, schématiquement et partiellement, un système d'analyse conforme à un exemple de mise en oeuvre de l'invention,
- la figure 2 représente une variante du système d'analyse de la figure 1,
- la figure 3 est un schéma en blocs visant à illustrer un exemple de fonctionnement du système d'analyse,
- la figure 4 représente des exemples de produits cosmétiques comportant un identifiant permettant de repérer le niveau de clarté,
- la figure 5 représente un exemple de support facilitant la sélection d'un produit en connaissant le niveau de clarté,
- la figure 6 représente le support de la figure 5 après avoir retourné l'un des volets supérieurs selon la flèche P de la figure 5,
- la figure 7 représente de manière plus détaillée le verso de deux volets pouvant se juxtaposer, et
- les figures 8 et 9 sont deux exemples de pages sur lesquelles sont portées des informations relatives à des fonds de teint pour deux gammes de produits adaptées respectivement à des niveaux de clarté différents.

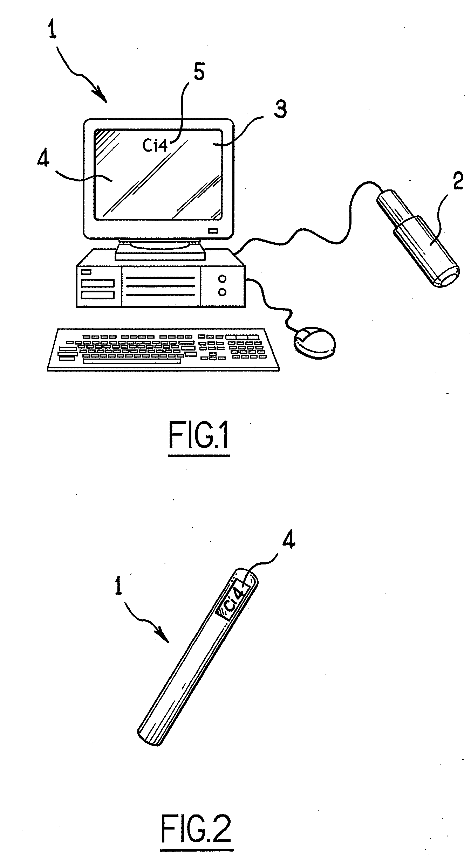

On a représenté à la figure 1, de façon schématique, un système d'analyse 1 réalisé conformément à l'invention, comportant une caméra 2 et des moyens de traitement 3 permettant de traiter les informations délivrées par la caméra, ce système de traitement se présentant dans l'exemple de la figure 1 sous la forme d'un micro-ordinateur comportant un écran 4.

Le micro-ordinateur peut être de tout type connu et comporte dans l'exemple considéré une carte d'acquisition graphique permettant de capturer les images délivrées par la caméra 2 afin de les traiter.

Le micro-ordinateur 3 est relié, le cas échéant, par un réseau informatique tel qu'Internet, à un serveur distant afin d'échanger des informations avec ce dernier, et notamment permettre au serveur distant d'enregistrer des informations liées aux images acquises et traitées par le micro-ordinateur.

La caméra 2 est de préférence une caméra couleur comportant un éclairage intégré et une optique grossissant plusieurs fois.

Le système d'analyse 1 est par exemple présent sur un point de vente ou dans un salon de beauté.

Le système d'analyse 1 peut encore être présent chez le consommateur lui-même. Dans ce cas, le traitement des images acquises par la caméra peut s'effectuer chez le consommateur ou à distance, après transmission à un serveur d'une image au moins de la peau.

Conformément à l'invention, le système d'analyse 1 est agencé pour délivrer après acquisition d'au moins une image un identifiant 5 qui se présente par exemple sous la forme d'un message alphanumérique s'affichant sur l'écran 4.

L'identifiant 5 peut être apposé par ailleurs sur les produits d'une gamme de produits cosmétiques afin de permettre au consommateur de repérer que ces produits sont bien adaptés au niveau de clarté correspondant. En variante, l'identifiant 5 n'est pas apposé sur les produits mais un moyen permettant de connaître les produits associés à un identifiant est prévu, par exemple sous la forme d'un support qui sera décrit plus loin.

De préférence, tous les niveaux de clarté que l'on peut rencontrer dans la population correspondent à un nombre d'identifiants assez peu élevé, par exemple compris entre 6 et 12, égal à 9 dans l'exemple considéré.

Bien entendu, le nombre d'identifiants pourra dépendre de la zone géographique et notamment du nombre de catégories ethniques différentes rencontrées au sein de celle-ci.

On peut ainsi proposer des identifiants en nombres différents aux consommateurs asiatiques, nord-américains et européens, par exemple.

Dans l'exemple considéré, les identifiants 5 se présentent chacun sous la forme de caractères alphanumériques comportant une ou plusieurs lettres et un numéro, les identifiants étant choisis par exemple parmi Ci1, Ci2, Ci3, ..., Ci8, Ci9.

On a représenté à la figure 2 une variante du système d'analyse 1 dans laquelle celui-ci se présente sous la forme d'un dispositif portable autonome comportant un afficheur 4, et une caméra et des moyens de traitement non apparents.

Ce système d'analyse peut en outre, le cas échéant, échanger des informations avec un ordinateur ou un terminal portable par une liaison sans fil, par exemple de type WI-FI® ou BLUETOOTH® .

Le système d'analyse peut encore comporter un terminal portable, notamment un téléphone portable, équipé d'une caméra et programmé de manière, après étalonnage, à traiter l'image acquise par la caméra de façon à afficher un identifiant correspondant au niveau de clarté de la peau.

Le cas échéant, le téléphone portable peut être associé durant l'acquisition de l'image à un dispositif optique comportant une source d'éclairage intégrée et une optique grossissante.

En variante encore, on peut utiliser un terminal portable pour acquérir une image de la peau et envoyer celle-ci par le réseau de téléphonie à un serveur distant agencé pour la traiter et délivrer en retour l'identifiant.

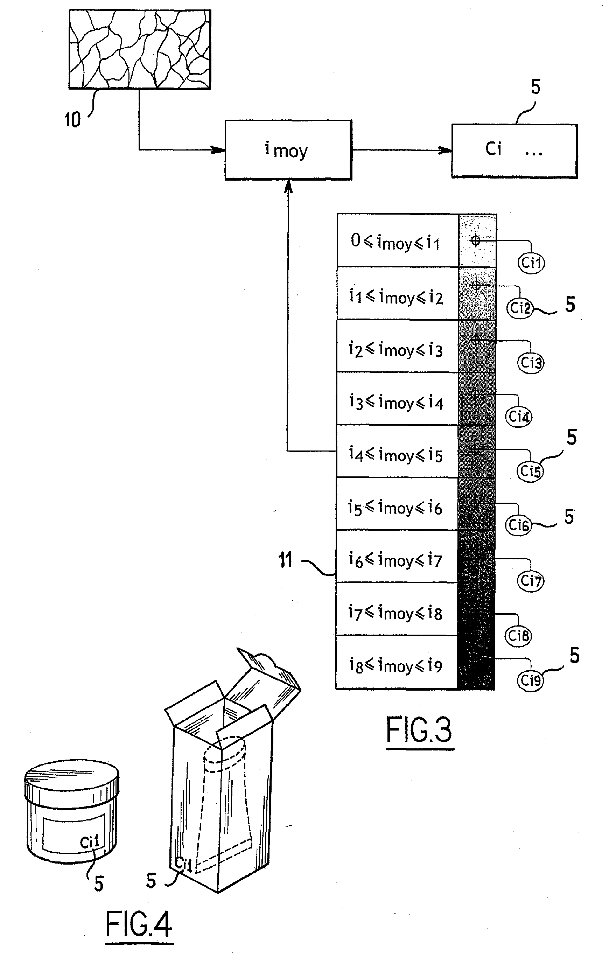

On va maintenant décrire en se référant à la figure 3 un exemple de fonctionnement du système d'analyse 1.

Une ou plusieurs images 10 sont acquises par la caméra 2 et le cas échéant enregistrées par le système d'analyse 1.

La ou les images en couleur sont transformées en images monochromes, par exemple en n'exploitant que le signal de vert. La caméra utilisée est sans balance automatique du blanc ou celle-ci a été désactivée.

Pour chaque image monochrome, une luminosité moyenne i_{moy} est déterminée et lorsque plusieurs images ont été acquises, une valeur moyenne des moyennes i_{moy} peut être calculée.

Par ailleurs, le système d'analyse 1 a en mémoire ou peut accéder à une base dans laquelle est enregistrée un tableau de correspondance 11 entre chaque identifiant 5 qu'il est possible d'afficher et une plage de valeurs de luminosité moyenne i_{moy}.

Ce tableau de correspondance 11 peut avoir été déterminé au préalable par exemple lors d'une phase d'étalonnage du système d'analyse 1, en présentant à la caméra 2 successivement un ensemble d'échantillons ayant des niveaux de clarté de référence.

En fonction de la luminosité moyenne i_{moy} mesurée et du tableau de correspondance 11, le système d'analyse 1 délivre l'identifiant correspondant.

Bien entendu, on ne sort pas du cadre de la présente invention lorsque le niveau de clarté est déterminé autrement, notamment lorsqu'un traitement plus complexe est effectué, sans passer par une image monochrome mais en utilisant par exemple tous les signaux couleur de l'image acquise par la caméra 2.

L'acquisition par la caméra 2 de plusieurs images peut être avantageuse en ce qu'elle permet des acquisitions sur différentes zones du visage ou du corps. Le système d'analyse peut notamment permettre d'effectuer par exemple une acquisition sur le front et les joues.

Chaque image acquise par la caméra 2 peut s'afficher sur l'écran 4 afin de permettre à l'opérateur de vérifier que l'image a été acquise dans de bonnes conditions.

Le cas échéant, on peut afficher sur l'écran 4 une mosaïque représentant toutes les images acquises par la caméra 2 et devant être traitées par le système d'analyse en vue de déterminer un niveau de clarté.

Le cas échéant, une fois le niveau de clarté déterminé, l'information correspondante peut être adressée par un réseau informatique ou de téléphonie à un serveur distant qui va mémoriser cette information ainsi que le nom ou un moyen permettant d'identifier la personne concernée, de façon à pouvoir proposer automatiquement ensuite des produits adaptés à cette personne.

Une fois l'identifiant 5 connu, le consommateur ou la personne qui le conseille voit le choix de produits adaptés à ce niveau de clarté facilité par l'existence, selon l'invention, de gammes de produits respectivement associées aux différents niveaux de clarté possibles.

L'utilisateur de niveau de clarté Ci1 peut par exemple porter son choix sur les produits de la gamme associée à ce niveau de clarté Ci1 et revêtus chacun de l'identifiant Ci1, comme on peut le voir sur la figure 4.

Un identifiant Ci... donné peut être apposé sur un produit correspondant de différentes façons, par exemple en étant imprimé sur l'emballage contenant le produit ou sur le récipient lui-même ou figurant sur une étiquette collée sur l'emballage ou sur le récipient. Le cas échéant, l'identifiant ne figure pas directement sur le produit et une table de correspondance peut par exemple être utilisée.

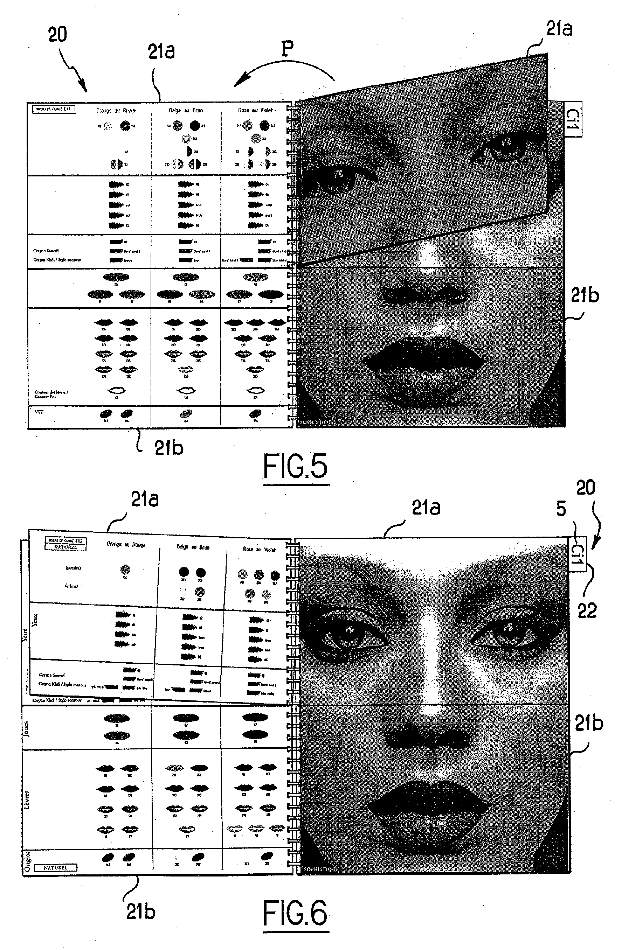

Pour aider le consommateur à faire son choix, un support 20 tel que celui représenté aux figures 5 et 6 peut lui être proposé.

Ce support 20 se présente par exemple sous la forme d'une brochure comportant un ensemble de feuilles et de volets reliés ensembles.

Cette brochure comporte des moyens de repérage permettant à l'utilisateur d'accéder rapidement à la gamme correspondant à son niveau de clarté. Dans l'exemple illustré, ces moyens de repérage comportent des onglets 22 sur chacun desquels est imprimé le niveau de clarté correspondant.

De préférence, afin de permettre à l'utilisateur de visualiser les effets du maquillage, le support 20 comporte des volets pouvant être juxtaposés pour représenter un visage complet et associés respectivement à différentes parties du visage, par exemple des volets supérieurs 21 a et des volets inférieurs 21b dans l'exemple considéré, les yeux et la partie supérieure du nez étant représentés sur le recto des volets supérieurs 21a et le bas du nez, les joues et les lèvres étant représentés sur le recto des volets inférieurs 21b.

Le verso des volets 21a et 21b comporte des informations concernant les produits utilisés lors du maquillage, dont le résultat est représenté sur le recto du volet suivant.

Ainsi, l'utilisateur peut, lorsque la brochure est ouverte, lire sur la page de gauche des informations qui correspondent au résultat de maquillage représenté sur la page de droite, et modifier à son gré le maquillage de la partie supérieure ou de la partie inférieure du visage en faisant tourner les volets correspondants.

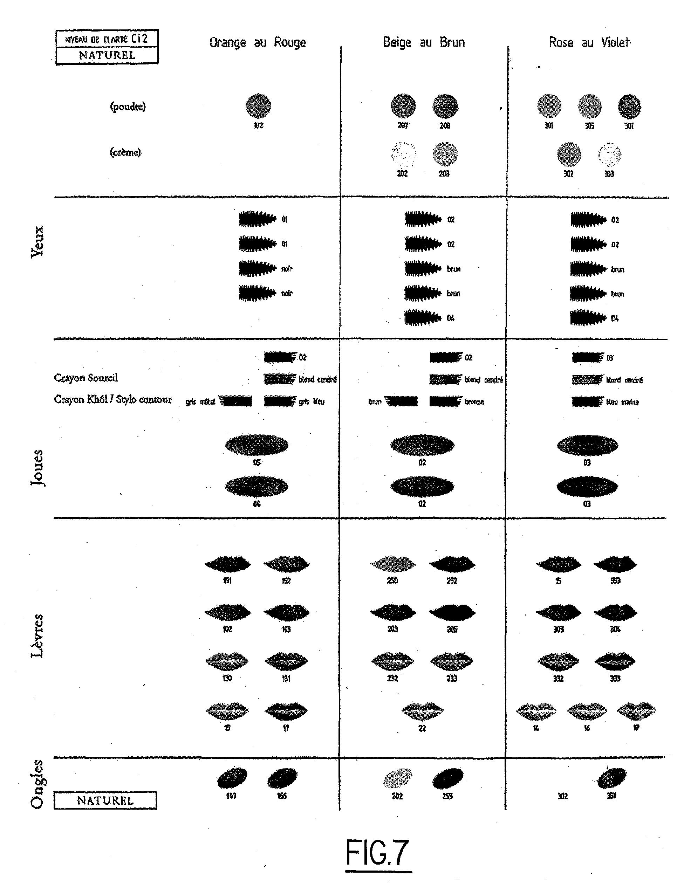

Des exemples d'informations figurant au verso de deux volets pouvant être juxtaposés ont été représentés à la figure 7.

Ces informations peuvent comporter par exemple des échantillons de la couleur des produits ainsi qu'un numéro de référence et une marque.

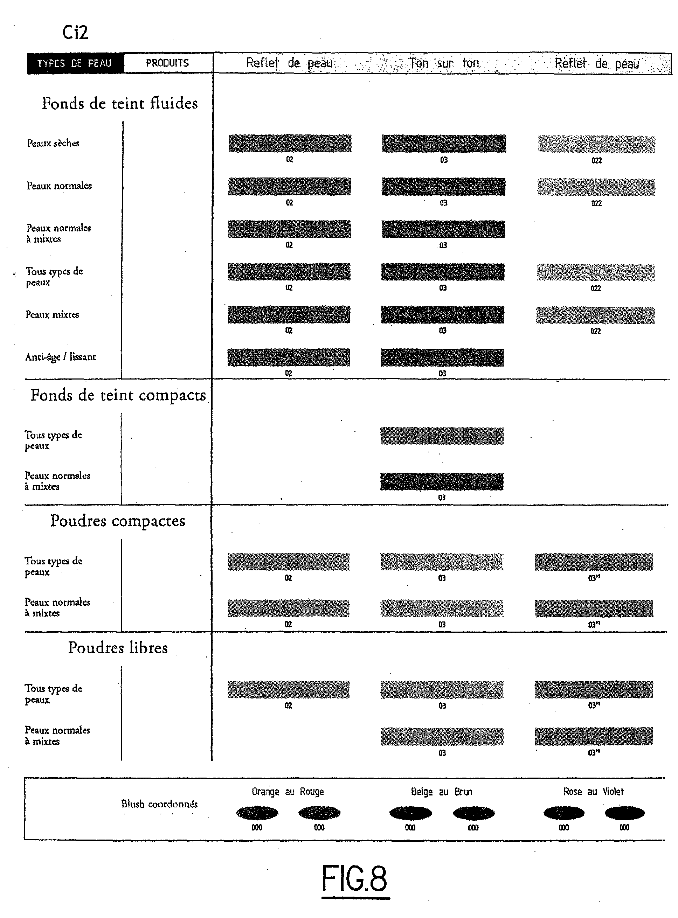

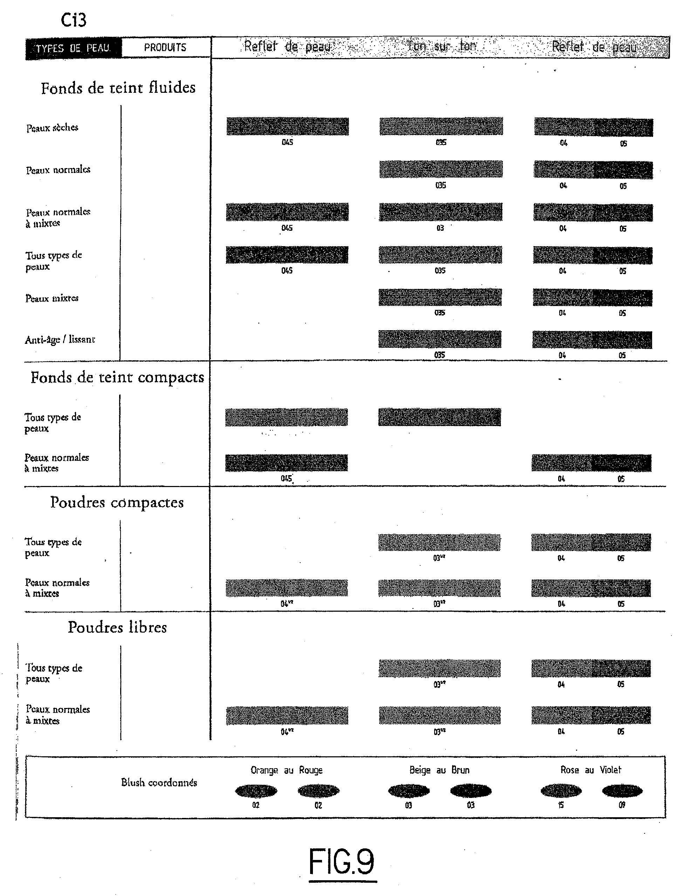

Le support 20 peut également comporter des pages dont des exemples sont représentés sur les figures 8 et 9.

On voit que peuvent figurer sur ces pages des informations liées à des familles de produits, par exemple des fonds de teint, en distinguant les formes galéniques, par exemple fluide, compacte, poudre compacte ou libre, les coloris disponibles pour chaque type de peau, par exemple peau sèche, peau normale, peau normale à mixte, tous types de peau, ou l'effet recherché, par exemple anti-âge/lissant. Les coloris peuvent être répertoriés en fonction de l'effet obtenu, par exemple bronzé, ton sur ton ou d'éclaircissement.

Le support 20 n'est pas limité à une forme papier et peut se présenter également sous forme logiciel, étant par exemple remis au consommateur après analyse de sa peau en étant enregistré sur un support informatique tel qu'un disque optique ou envoyé à celui-ci sous la forme d'un fichier par messagerie électronique.

Bien entendu, l'invention n'est pas limitée aux exemples qui viennent d'être décrits et la mesure du niveau de clarté de la peau peut notamment se combiner avec d'autres analyses de la peau, notamment des mesures de son hydratation, du taux de sébum, etc.

Dans toute la description, l'expression « comportant un » doit être comprise comme étant synonyme de « comportant au moins un », sauf si le contraire est spécifié.

## Revendications

1. Ensemble comportant :
- une pluralité de gammes de produits cosmétiques, chaque gamme regroupant un ensemble de produits associés à un même niveau de clarté de la peau, ce niveau de clarté étant repéré par un identifiant (5),
- un système d'analyse (1) d'au moins une image de la peau, agencé pour délivrer un identifiant (5) représentatif du niveau de clarté de la peau analysée, cet identifiant étant l'un de ceux desdites gammes de produits.

2. Ensemble selon la revendication 1, **caractérisé par le fait que** le système d'analyse comporte une caméra (2), de préférence une caméra couleur, permettant d'acquérir au moins une image de la peau avec un grossissement optique, notamment un facteur de grossissement compris entre 3 et 15.

3. Ensemble selon la revendication 2, **caractérisé par le fait que** la caméra comporte une source d'éclairage intégrée, notamment une source de lumière blanche.

4. Ensemble selon la revendication 1, **caractérisé par le fait que** la caméra est sans balance du blanc automatique activée.

5. Ensemble selon l'une des revendications 2 et 3, **caractérisé par le fait que** la caméra est agencée de manière à pouvoir être appliquée contre la peau.

6. Ensemble selon l'une quelconque des revendications 1 à 4, **caractérisé par le fait que** le système d'analyse comporte des moyens agencés pour convertir, le cas échéant, l'image délivrée par la caméra en une image monochrome et déterminer la luminosité moyenne d'une partie au moins de l'image monochrome.

7. Ensemble selon la revendication 6, **caractérisé par le fait que** l'image monochrome est une image vert et noir.

8. Ensemble selon l'une quelconque des revendications précédentes, **caractérisé par le fait que** le système d'analyse comporte un micro-ordinateur équipé d'une carte d'acquisition graphique et programmé de manière à calculer une luminosité moyenne à partir d'une image au moins transmise par une caméra.

9. Ensemble selon la revendication 8, **caractérisé par le fait que** le système d'analyse est agencé pour affecter à la valeur de luminosité moyenne un niveau de clarté, en utilisant notamment une table de correspondance (11), dans laquelle à chaque niveau de clarté est associée une plage de valeur de luminosité moyenne.

10. Ensemble selon l'une quelconque des revendications précédentes, **caractérisé par le fait que** le nombre de niveaux de clarté est supérieur à 2, et notamment est compris entre 3 et 25, étant de préférence compris entre 6 et 12, notamment égal à 9.

11. Ensemble selon l'une quelconque des revendications précédentes, **caractérisé par le fait que** le système d'analyse est agencé pour délivrer une information relative à la tonalité de couleur de la peau, notamment une indication matérialisant la tonalité plutôt jaune ou plutôt rouge de la peau.

12. Ensemble selon l'une quelconque des revendications 1 à 11, **caractérisé par le fait qu'**il comporte au moins un support (20) destiné à faciliter le choix d'un produit, connaissant le niveau de clarté de la peau.

13. Ensemble selon la revendication 12, **caractérisé par le fait que** le support est agencé de manière à permettre au consommateur de visualiser une pluralité de pages contenant des informations associées aux produits desdites gammes.

14. Ensemble selon l'une des revendications 12 et 13, **caractérisé par le fait que** le support se présente sous la forme d'une brochure comportant des feuilles reliées entre elles et des onglets (22) sur lesquels sont imprimés les différents identifiants.

15. Ensemble selon l'une quelconque des revendications 12 à 14, **caractérisé par le fait que** le support comporte des volets (21a, 21b) qui correspondent à des parties respectives du visage et qui permettent de modifier l'aspect du maquillage d'une partie seulement du visage tout en laissant le maquillage inchangé pour la ou les autres parties du visage correspondant aux autres volets.

16. Ensemble selon la revendication 14, **caractérisé par le fait que** le support (20) comporte des volets correspondant à des demi-visages, notamment des volets supérieurs (21a) correspondant au maquillage des yeux et des volets inférieurs (21b) correspondant au maquillage des lèvres et des joues.

17. Ensemble selon l'une des revendications 14 et 15, **caractérisé par le fait que**, pour un niveau de clarté au moins, le support comporte au moins deux volets successifs correspondant à la même partie du visage, chacun de ces volets comportant au recto une image du visage et au verso des informations concernant des produits de maquillage des yeux ou des joues, les volets étant agencés de telle sorte que les produits référencés sur le verso d'un volet correspondent au résultat de maquillage présenté sur le recto du volet immédiatement suivant.

18. Ensemble selon la revendication 12, **caractérisé par le fait que** le support est un logiciel programmé pour permettre l'affichage simultané sur une même page écran d'une simulation de maquillage et du ou des produits utilisés pour cette simulation.

19. Ensemble selon l'une quelconque des revendications 1 à 17, **caractérisé par le fait que** chaque gamme de produits comporte des produits destinés à être appliqués sur la peau, comme des fonds de teint, ombres à paupières, eye-liner, blush, des produits destinés à être appliqués sur les muqueuses tels qu'un rouge à lèvres, crayon à lèvres, des produits destinés à être appliqués sur les cils ou les sourcils tels qu'un mascara, un crayon à sourcils, un crayon khôl, et des produits destinés à être appliqués sur les ongles tels qu'un vernis à ongles.

20. Système d'analyse de la clarté de la peau, comportant :
- une caméra (2) permettant d'acquérir au moins une image de la peau,
- des moyens de traitement (3) d'au moins une image acquise par la caméra, agencés pour délivrer un identifiant (5) associé au niveau de clarté mesuré, cet identifiant figurant par ailleurs
- sur des produits d'une pluralité de gammes de produits associées respectivement aux différents niveaux de clarté susceptibles d'être délivrés par les moyens de traitement, ou
- sur un support contenant des références de produits d'une pluralité de gammes de produits associées respectivement aux différents niveaux de clarté susceptibles d'être délivrés par les moyens de traitement.

21. Procédé de traitement cosmétique, notamment de maquillage de la peau, des phanères ou des muqueuses, comportant les étapes suivantes :
- associer à un consommateur un identifiant (5) permettant de repérer le niveau de clarté de sa peau,
- en fonction de cet identifiant, sélectionner au moins un produit cosmétique parmi un ensemble de gammes de produits repérées par des identifiants de niveau de clarté,
- maquiller le consommateur avec le ou les produits sélectionnés.

22. Procédé de fabrication d'un ensemble de gammes de produits cosmétiques, notamment des produits destinés au maquillage de la peau, des muqueuses et des phanères, comportant l'apposition sur chaque produit de la gamme d'un identifiant (5) correspondant à un niveau de clarté de la peau pour lequel ce produit est adapté.

23. Procédé de fabrication d'un ensemble de gammes de produits cosmétiques, notamment des produits destinés au maquillage de la peau, des muqueuses et des phanères, comportant l'attribution à chaque produit de la gamme d'un identifiant correspondant à un niveau de clarté de la peau pour lequel ce produit est adapté, l'attribution de l'identifiant se faisant en portant au moins une référence, par exemple commerciale, du produit dans une table en correspondance avec un identifiant de niveau de clarté, la table étant un document de type papier ou un ficher informatique.
